# FASCICULE DE BREVET EUROPEEN

(11) **EP 2 583 695 B1**
(45) Date de publication et mention de la délivrance du brevet: **21.05.2014**
(21) Numéro de dépôt: 12187357.4
(22) Date de dépôt: 05.10.2012
(51) Int. Cl.: A61L 2/18, A61L 2/08

(54) **Procédé de préparation d'un emballage stérile d'une solution aqueuse d'acide peracétique, par irradiation gamma de la surface dudit emballage**
Verfahren zur Vorbereitung einer sterilen Verpackung einer Peressigsäure Lösung durch Gammabestrahlung der Oberfläche dieser Verpackung
Method for preparing a sterile package of a peracetic acid solution, by gamma irradiation of the surface of said package

(30) Priorité: 21.10.2011 FR 1159564
(43) Date de publication de la demande: 24.04.2013
(73) Titulaire: Bioxal, 75007 Paris (FR)
(72) Inventeur: DESMURS, MARIE-JEANNE, 71460 SAINT GENGOUX LE NATIONAL (FR); MABILLE, JEAN-BENOIT, 71100 LA CHARMEE (FR); PALMA, LUCIE, 71100 LUX (FR); STEINHAUER, Udo, 71103 CHALON SUR SAÔNE (FR)
(74) Mandataire: Conan, Philippe Claude

(56) Documents cités:
- EP-A1- 0 873 687
- FR-A1- 2 756 259
- FR-A1- 2 855 759
- US-A1- 2004 234 569
- US-A1- 2010 196 505

## Description

Les solutions aqueuses d'acide peracétique prêtes à l'emploi sont largement connues et commercialisées pour désinfecter notamment des matériels médico-chirurgicaux et notamment ceux qui ne supportent pas stérilisation à chaud. De telles solutions ont ainsi été développées pour désinfecter les endoscopes, les lentilles de contact ou le petit matériel de dentisterie ou d'ophtalmologie. Elles sont par exemple décrites dans la demande de brevet européen EP 0 873 687 A1 et dans la demande de brevet français 2 855 759.

Cependant, une des conditions nécessaires à un bon pouvoir désinfectant, réside dans la qualité de la solution aqueuse elle-même et y compris de son emballage. Il faut donc en limiter, voire en supprimer son pouvoir pathogène. Un des moyens d'obtention d'emballages stériles consiste à les soumettre à une irradiation aux rayons gamma, par exemple comme divulguée dans la demande de brevet français 2756 259, ou à une irradiation ultra-violette comme divulguée dans la demande de brevet américain US 2004/0234569 A1.

Or, les solutions aqueuses d'acide peracétique sont réputées relativement instables notamment au rayonnement ultraviolet et leur conservation doit se faire à l'abri de la lumière, pour en limiter leur décomposition. De plus de telles compositions désinfectantes sont aussi connues pour être instables lors d'une exposition au rayonnement gamma, ainsi qu'il l'est divulgué dans la demande de brevet américain US 2010/0196505 A1.

Enfin on sait que l'irradiation du peroxyde d'hydrogène conduit à la formation de radicaux libres HO₂˙ or HO˙ et donc à la décomposition du peroxyde.

Les inventeurs ont ainsi cherché à développer une méthode de décontamination qui ne dégrade la solution désinfectante.

C'est pourquoi, selon un premier aspect, l'invention a pour objet un procédé de préparation d'un emballage stérile d'une solution aqueuse d'acide peracétique par décontamination de la surface dudit emballage, **caractérisé** **en ce** qu'il comprend :
- Une étape a) au cours de laquelle ladite solution aqueuse d'acide peracétique comprenant pour 100% massique de :
- De 0,01 % massique à 5% massique d'acide peracétique,
- De 1 % massique à 10% massique de peroxyde d'hydrogène,
- Et la quantité d'acide acétique nécessaire pour maintenir l'équilibre chimique de ladite solution, est introduite dans ledit emballage pour obtenir ledit emballage contenant ladite solution aqueuse d'acide peracétique ;
- Une étape b) au cours de laquelle ledit emballage contenant ladite solution aqueuse d'acide peracétique est, à l'issue de l'étape a), fermé au moyen d'un dispositif muni d'un évent ;
- Une étape c) au cours de laquelle ledit emballage fermé contenant ladite solution aqueuse d'acide peracétique, issu de l'étape b), est exposé à un rayonnement gamma à une dose de rayonnement comprise entre 5 000 Gray et 30 000 Gray, et plus particulièrement jusqu'à 28 000 Gray.

Par quantité d'acide nécessaire à maintenir l'équilibre chimique de ladite solution aqueuse d'acide peracétique, on signifie que la concentration en acide acétique est généralement comprise entre 0,1% massique et 45% massique.

La solution aqueuse d'acide peracétique mise en oeuvre dans le procédé tel que défini ci-dessus peut comprendre des produits auxiliaires tels que :
- De 0,01 % massique à 5% massique d'un ou de plusieurs agents inhibiteurs de corrosion comme les sels alcalins ou alcalinoterreux de l'acide phosphorique, par exemple l'hydrogénophosphate de sodium ou le dihydrogénophosphate de sodium ;
- De 0,001% massique à 5% massique d'un ou de plusieurs agents stabilisants et/ou séquestrant de radicaux libres, par exemple, l'acide chlorhydrique, l'acide sulfurique, l'acide phosphorique, le pyrophosphate de sodium, l'acide dipicolinique les acides phospohiniques, ou les butyl hydroxy toluènes ;
- De 0,0001 % massique à 0,05% massique d'un ou de plusieurs agents de coloration compatibles avec l'acide peracétique ;
- De 0,001% massique à 1% massique d'un ou de plusieurs oxydes d'amine gras, par exemple l'oxyde de coodiméthylamine, l'oxyde de tétradécyl diméthylamine ou l'oxyde de diméthyl stéaryl amine ;
- De 0,001 % massique à 0,5% massique Un ou plusieurs tensioactifs non ioniques, par exemple un alcool gras polyéthoxylé et/ou polypropoxylé comme le Génapol™2908D qui est un mélange d'alcools en C11, C13 et C15 (6 à 70E, 30P).

Selon un aspect particulier du procédé tel que défini précédemment, la solution aqueuse d'acide peracétique mise en oeuvre comprend pour 100% massique de 0,05% massique à 1 % massique d'acide peracétique,

Selon un aspect particulier du procédé tel que défini précédemment, la solution aqueuse d'acide peracétique mise en oeuvre comprend pour 100% massique de 1 % massique à 5% massique de peroxyde d'hydrogène,

Comme exemple de composition mise en oeuvre dans le procédé tel que défini précédemment, il y a par exemple une composition consistant en, pour 100% massique :
- De 0,01 % massique à 5% massique d'acide peracétique,
- De 2% massique à 5% massique de peroxyde d'hydrogène,
- La quantité d'acide acétique nécessaire pour maintenir l'équilibre chimique de ladite solution,
- De 0,5% massique à 1 % massique d'un agent stabilisant et
- Le complément à 100% massique en eau.

Par emballage, on désigne dans le cadre du procédé tel que défini ci-dessus, notamment un pulvérisateur manuel de type «Airless™» de volume compris entre 0,1 litre et 5 litres, de préférence entre 0,5 litre et un litre.

Selon un aspect particulier du procédé tel que défini ci-dessus, ledit emballage fermé contenant ladite solution aqueuse d'acide peracétique, issu de l'étape b), est soumis à une étape d) au cours de laquelle il est emballé dans un double sac muni d'un évent avant la mise en oeuvre de l'étape c).

Dans le procédé tel que défini ci-dessus l'étape d'irradiation par rayon gamma est réalisée avec un appareil disponible commercialement comprenant une source de Cobalt 60 émettant ledit rayonnement gamma. La durée d'exposition à ce dit rayonnement gamma est généralement comprise entre 2 heures et 6 heures.

Dans un mode de réalisation préféré, ledit contenant est placé dans un sac en matériau dit Tivek™.

Les inventeurs ont constaté que de façon inattendue, une solution aqueuse d'acide peracétique prêt à l'emploi, consistant en pour 100% de sa masse :
- De 0,01 % massique à 5% massique d'acide peracétique,
- de 2% massique à 5% massique de peroxyde d'hydrogène,
- la quantité d'acide acétique nécessaire pour maintenir l'équilibre chimique de ladite solution,
- de 0,5% massique à 1 % massique d'un agent stabilisant et
- le complément à 100% massique en eau,
contenue dans un contenant ayant subi une irradiation de 15 000 Gray n'avait, après conservation pendant un an à l'abri de la lumière, subie aucune décomposition, contrairement à ce que l'on pouvait attendre.

## Revendications

1. Procédé de préparation d'un emballage stérile d'une solution aqueuse d'acide peracétique par décontamination de la surface dudit emballage, **caractérisé en ce qu'**il comprend :
- Une étape a) au cours de laquelle ladite solution aqueuse d'acide peracétique comprenant pour 100% massique de :
- De 0,01 % massique à 5% massique d'acide peracétique,
- De 1 % massique à 10% massique de peroxyde d'hydrogène,
- Et la quantité d'acide acétique nécessaire pour maintenir l'équilibre chimique de ladite solution,
est introduite dans ledit emballage pour obtenir ledit emballage contenant ladite solution aqueuse d'acide peracétique ;
- Une étape b) au cours de laquelle ledit emballage contenant ladite solution aqueuse d'acide peracétique est, à l'issue de l'étape a), fermé au moyen d'un dispositif muni d'un évent ;
- Une étape c) au cours de laquelle ledit emballage fermé contenant ladite solution aqueuse d'acide peracétique, issu de l'étape b), est exposé à un rayonnement gamma à une dose de rayonnement comprise entre 5 000 Gray et 30 000 Gray, et plus particulièrement jusqu'à 28 000 Gray.

2. Procédé tel que défini à la revendication 1, dans lequel ledit emballage fermé contenant ladite solution aqueuse d'acide peracétique, issu de l'étape b), est soumis à une étape d) au cours de laquelle il est emballé dans un double sac muni d'un évent avant la mise en oeuvre de l'étape c).

## Patentansprüche

1. Verfahren zum Herstellen einer sterilen Verpackung für eine wässrige Peressigsäure-Lösung durch Dekontamination der Oberfläche der Verpackung, **dadurch gekennzeichnet, dass** es Folgendes umfasst:
- einen Schritt a), im Laufe dessen die wässrige Peressigsäure-Lösung, die pro 100 Massen-% Folgendes enthält:
- 0,01 Massen-% bis 5 Massen-% Peressigsäure,
- 1 Massen-% bis 10 Massen-% Wasserstoffperoxid,
- und die erforderliche Menge Essigsäure, um das chemische Gleichgewicht der Lösung aufrechtzuerhalten,
in die Verpackung eingebracht wird, um die Verpackung zu erhalten, welche die wässrige Peressigsäure-Lösung enthält;
- einen Schritt b), im Laufe dessen die Verpackung, welche die wässrige Peressigsäure-Lösung enthält, am Ende von Schritt a) mit Hilfe einer mit einem Luftloch versehenen Vorrichtung verschlossen wird;
- einen Schritt c), im Laufe dessen die verschlossene Verpackung, welche die wässrige Peressigsäure-Lösung enthält und aus Schritt b) stammt, einer Gammastrahlung mit einer Strahlendosis zwischen 5.000 Gray und 30.000 Gray, und insbesondere bis zu 28.000 Gray, ausgesetzt wird.

2. Verfahren nach Anspruch 1, bei dem die verschlossene Verpackung, welche die wässrige Peressigsäure-Lösung enthält und aus Schritt b) stammt, einem Schritt d) unterzogen wird, im Laufe dessen sie vor der Durchführung von Schritt c) in einem doppelten mit einem Luftloch versehenen Beutel verpackt wird.

## Claims

1. Method for preparing a sterile packaging for an aqueous peracetic acid solution by decontaminating the surface of said packaging, **characterised in that** it comprises:
- a step a) during which said aqueous peracetic acid solution, comprising, for 100 % by mass:
- from 0.01 % by mass to 5 % by mass peracetic acid,
- from 1 % by mass to 10 % by mass hydrogen peroxide,
- and the amount of acetic acid required to maintain the chemical equilibrium of said solution,
is introduced into said packaging to obtain said packaging containing said aqueous peracetic acid solution;
- a step b) during which said packaging containing said aqueous peracetic acid solution is, at the end of step a), closed by means of a device equipped with a vent;
- a step c) during which said closed packaging from step b) and containing said aqueous peracetic acid solution is exposed to gamma radiation at a radiation dose of between 5,000 gray and 30,000 gray, and more particularly of up to 28,000 gray.

2. Method as defined in claim 1, wherein said closed packaging from step b) and containing said aqueous peracetic acid solution is subjected to a step d) during which it is wrapped in a double bag equipped with a vent before step c) is carried out.
